# FASCICULE DE BREVET EUROPEEN

(11) **EP 3 165 100 B1**
(45) Date de publication et mention de la délivrance du brevet: **13.12.2017**
(21) Numéro de dépôt: 16194916.9
(22) Date de dépôt: 20.10.2016
(51) Int. Cl.: A23L 33/105, A23L 29/25, A23L 29/256

(54) **GALÉNIQUE SOLIDE**
FESTE GALENISCHE FORM
SOLID GALENIC FORM

(30) Priorité: 05.11.2015 BE 201505720
(43) Date de publication de la demande: 10.05.2017
(73) Titulaire: LOËN HOLDING S.A.R.L., 2330 Luxembourg (LU)
(72) Inventeur: COURTOT, Lionel Roland, 01800 Perouges (FR)
(74) Mandataire: Donné, Eddy

(56) Documents cités:
- RO-A2- 96 596
- US-A1- 2013 059 038
- US-B1- 8 722 109

## Description

L'invention concerne des compléments alimentaires sous forme solide, renfermant des actifs naturels à effet thérapeutique et/ou nutritif, et gastro-résistants, destinés à la consommation humaine ou animale.

Plus spécialement, l'invention a pour but de délivrer des compléments alimentaires contenant des extraits de plantes, d'huiles essentielles et d'autres ingrédients naturels.

Il s'agit de matières à goût trop amer, répugnant, trop acide, trop métallique ou encombrant pour permettre une consommation de ces matières dans leur état natif ou naturel.

Des exemples de ces matières sont les huiles essentielles chémotypées, extraits de plantes médicinales fraîches sous forme liquide, extraits de plantes médicinales sèches (écorces, racines,...) sous forme de poudre, des vitamines, des minéraux, des huiles végétales, etc...

Souvent ces matières sont administrées sous forme liquide. Une telle galénique liquide est totalement adaptée par sa nature, son odeur et goût aux habitudes alimentaires des herbivores, mais elle devient une vraie barrière pour le traitement des omnivores et carnivores qui sont totalement repoussés par les caractéristiques olfactives et gustatives de ces produits et qui en plus peuvent souffrir de gênes sur leur appareil digestif.

Afin d'éviter les désavantages d'une galénique liquide, il convient de transformer les aliments complémentaires dans un état plus solide comme par une gélification ou solidification contrôlée.

Pour maintenir la conservation entière des actifs il faut travailler les mêmes formulations et matières premières, et ceci à froid. US 2013/059038 A1 décrit une composition qui contient des extraits de plantes (extraits de thé noir), des huiles essentielles et des gommes végétales (Acacia) et RO 96 596 A2 décrit des comprimés qui contiennent des extraits de plantes et des huiles essentielles (camomille). En cherchant un procédé utilisable, l'analyse des composants des formulations de produits naturels a été entreprise pour identifier les composants favorisant ou défavorisant la gélification.

Il en résulte que le composant favorisant la gélification est l'eau et que les composants pouvant nuire à la gélification sont l'alcool et les huiles essentielles qui plutôt favorisent la précipitation ou le déphasage, qui par définition, désunissent les différents composants.

Les produits suivants sont des gélifiants connus :
- la gélatine de porc ;
- les gommes arabiques ;
- l'agar agar ;
- les alginates.

La gélatine de porc est exclue comme étant d'origine non végétale.

Les gommes arabiques donnent un épaississement des produits ciblés, mais ceux-ci sont restés systématiquement sous forme pâteuse sans jamais se solidifier.

L'agar agar, bien que performant présente l'inconvénient d'impliquer un procédé à chaud, atteignant une température de 90 °C, ce qui élimine en grande partie l'alcool et les huiles essentielles et par conséquent aussi une grande partie des actifs naturels.

Afin d'obtenir un produit final de la même consistance qu'un bonbon gélatineux conventionnel mais sans perdre l'activité de ses composants d'origine naturelle, la méthode suivante a prouvé être efficace dans nos expériences.

### Etape I : Composition de base

Dans une première étape une composition de base est obtenue, composée de :
1- extraits de plantes sous forme liquide concentrés en principes actifs (phytothérapie) : entre 20 et 70 % ;
2- Huiles essentielles (aromathérapie) :
   entre 1 et 15 % ;
3- Eau :
   entre 20 et 60 % ;
Optionnel :
4- Extraits de plantes sous forme de poudres concentrés en principes actifs (phytothérapie) ;
5- Minéraux (oligothérapie);
6- Vitamines ;
7- Huiles végétales ;
8- Plasma marin ;
9- Silicium organique (antiagglomérant).

Pour l'extrait de plante, des plantes médicinales peuvent être utilisées sous forme de :
- extraits hydro-alcooliques, obtenus par extraction ;
- extraits hydro-alcooliques glycérinés, obtenus par extraction ;
- teintures mères, obtenu par extraction ;
- macérats glycérinés de bourgeons, obtenus par extraction ;

Pour l'extrait de plante servant à la phytothérapie, il peut être dérivé de toutes plantes possibles, telles que :
- *Alchemilla vulgaris* HAG, la partie utilisée étant l'aérienne fleurie (origine : France) ;
- *Gi nkgo biloba* HA ;
- *Melilotus* TM ;
- *Cichorium intybus*, la partie utilisée sous forme de poudre étant la racine (origine : France) ;

Les huiles essentielles sont obtenues par distillerie des plantes aromatiques.

Pour l'aromathérapie, toutes huiles essentielles peuvent être utilisées, comme par exemple :
- *Citrus limomum*, la partie utilisée étant le zeste exprimé (origine : Italie) ;
- *Ocimum Basilicum*, la partie utilisée étant la plante fleurie (origine : Vietnam) ;
- *Mentha piperita,* la partie utilisée étant la plante fleurie (origine : Inde).

Les huiles végétales peuvent être toutes huiles possibles, telles que :
- *Ricinus communis ;*
- *Vitis vinifera ;*
- *Corylus avellana.*

Les oligoéléments peuvent être tous minéraux possibles, tels que :
- le zinc (Zn)
- le silicium (Si)
- le magnésium (Mg).

### Etape II : Prémix solidifiant et apportant la gastro-résistance.

Dans une deuxième étape un prémix solidifiant est ajouté, composé de :
- Alginates (exemple : algues brunes E401) et Calcium (sulfate de calcium) à effet gélifiant et apportant la gastro-résistance du produit ;
- Gomme(s) végétale(s) à effet émulsionnant, liant, épaississant (exemples : gomme Karaya, Sterculia, Acacia à gomme ou Acacia senegal gum extrait, etc...):
Optionnel :
- Substances naturelles ou chimiques à effet retardant (exemples : chélateurs d'eau comme le bambusa bambou, chélateurs de calcium comme les polyphosphates, monosaccharides, etc...) ;
- Plante sèche hydrophile (exemple : Plantage ovata) ;
- Substances naturelles ou chimiques à effet anti-moussant et antiagglomérant (exemple : dioxyde de silicium E551) ;
- Aromatisants naturels ou chimiques (exemple : la vanille) ;
- Colorants alimentaires naturels ou chimiques (exemple : la betterave) ;
- Autres substances, même chimiques, apportant aussi la gastro-résistance des actifs.

Ce procédé a résulté dans des compléments alimentaires sous forme solide, et restant secs, tandis que la valeur nutritive et thérapeutique des substances végétales (actifs naturels) était conservée grâce au prémix solidifiant ajouté à température ambiante.

Un avantage du procédé décrit est qu'il permet d'offrir des compléments alimentaires gastro-résistants pour consommation humaine ou animale, qui ne nuisent plus aux goûts du consommateur à cause de leurs caractéristiques olfactives et gustatives et qui ne sont plus gênants pour son appareil digestif.

Un autre avantage du procédé décrit est qu'il permet de conserver des compléments alimentaires sous forme sèche et solide et sans réfrigération ce qui facilite le transport et le dépôt de ces produits.

Pour plus de clarté, quelques exemples de réalisation de compléments alimentaires sont donnés, référence étant faite aux dessins annexés dans lesquels :
La figure 1 représente schématiquement les étapes du procédé de production selon l'invention ;
La figure 2 est une vue en perspective d'un complément alimentaire formé par le procédé de l'invention ;
La figure 3 est une vue en perspective d'un autre complément alimentaire formé par le procédé de l'invention.

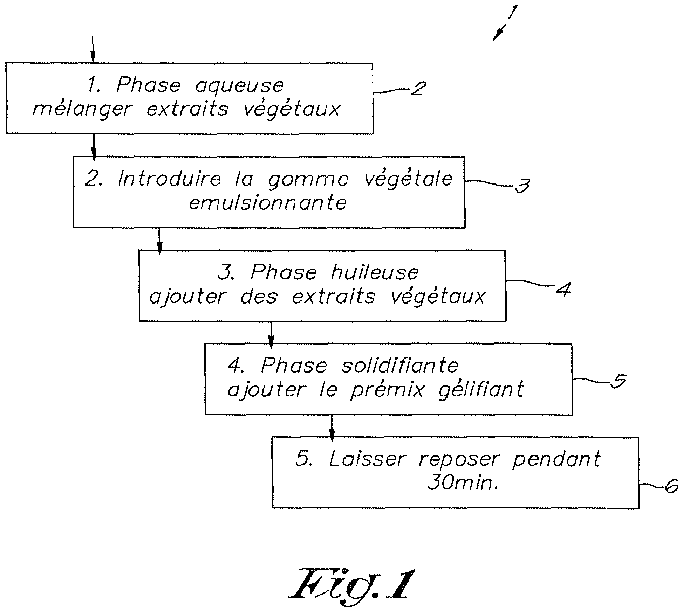

Dans la figure 1 les phases consécutives du procédé de production de compléments alimentaires 1 selon l'invention sont représentés comprenant :
1. La phase aqueuse 2 dans laquelle les extraits végétaux sont mélangés ;
2. L'introduction d'une gomme végétale 3 émulsionnante ;
3. La phase huileuse 4 introduisant des extraits végétaux sous forme d'huiles ;
4. La phase solidifiante 5 avec l'introduction du prémix gélifiant, pour émulsionner jusqu'à obtenir une pâte ;
5. La phase de repos 6 , laissant agir le prémix solidifiant jusqu'à obtenir un solide stable.

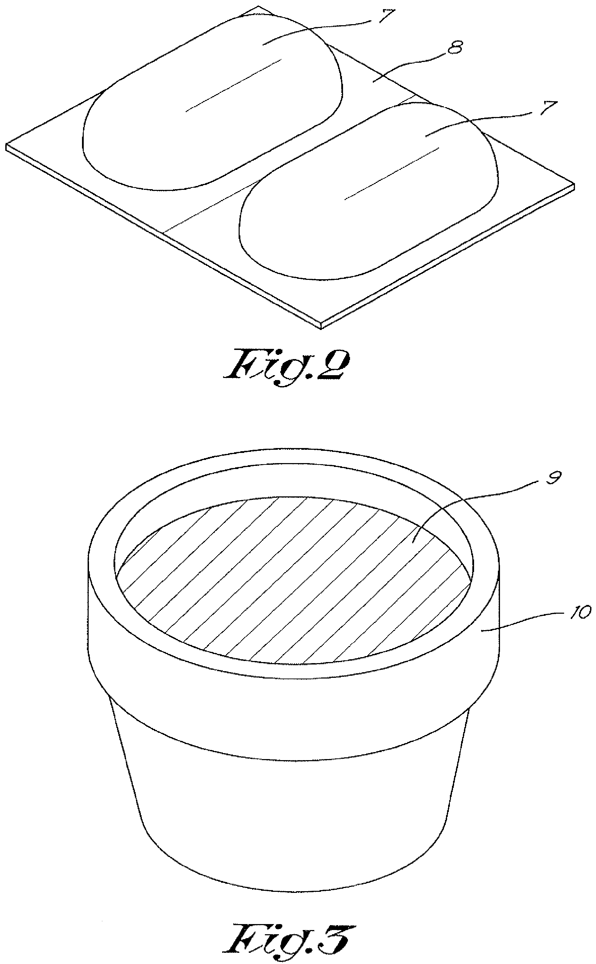

Dans la figure 2 un complément alimentaire formé par le procédé de l'invention est montré, ayant la forme d'une gélule individuelle 7 sous blister 8 bien que des gélules en vrac peuvent aussi être délivrées dans un pot par exemple.

Dans la figure 3 un autre exemple d'un complément alimentaire formé par le procédé de l'invention est montré, ayant la forme d'une pâte 9 dans un pot 10.

Il est évident que le complément alimentaire peut se présenter sous n'importe quelle forme, comme dans une seringue, des bonbons, etc...

Il en suit qu'un complément alimentaire, obtenu par le procédé décrit peut être utilisé pour le traitement préventif des hommes ou des animaux par voie orale, comme le procédé de production est capable de préserver les substances naturelles des plantes sans perdre leur valeur nutritive et thérapeutique en étant limité à des températures ambiantes.

Afin d'illustrer le procédé de production selon l'invention, l'application du procédé sur la production d'un complément alimentaire étant en particulier un produit à visée de « drainage » est décrit comme un exemple dans ce qui suit.
1) Mélanger dans la phase aqueuse :
   Eau : 35 %
   Plasma marin : 10 %
   Silicium colloïdale : 5 %
   Extrait* de parties aériennes d'ortie piquante (urtica doioica) : 10 %
   Extrait* de racine de pissenlit (*Taraxacum officinalis*) : 15 %
   Extrait* de feuilles et racines d'artichaut (*Cynara scolymus*) : 10 %
   * : *extraits hydro-alcoolique glycériné* ou *hydro-alcoolique.*
2) Introduire la gomme arabique et émulsionner :
   Gomme arabique : 1 %
3) Continuer à émulsionner la phase aqueuse, tout en introduisant la phase huileuse :
   Huile essentielle d'eucalyptus (*Eucalyptus globulus*) : 2 %
   Huile essentielle d'orange amère (*Citrus aurantium*) : 1 %
   Huile essentielle de cyprès (*Cupressus sempervirens*) : 1 %
4) Dans la phase solidifiante, introduire le prémix gélifiant et les plantes en poudre préparés au préalable, puis mélanger.
   Poudre de chlorelle (*Chlorella pyrenoidosa*) : 2 %
   Alginate de sodium : 7,8 %
   Sulfate de calcium : 0,1 %
   Gomme xanthane : 0,1 %
5) Laisser reposer jusqu'à solidification (environ 30 minutes).

Il est évident que l'invention n'est nullement limitée aux exemples décrits ci-avant mais que de nombreuses modifications peuvent être apportées à la forme et à la composition des compléments alimentaires tout en respectant le procédé de production décrit ci-avant sans sortir du cadre de l'invention telle que définie dans les revendications suivantes.

## Revendications

1. Procédé de préparation pour compléments alimentaires, **caractérisé en ce qu'**il comprend au moins les étapes suivantes :
Dans une première étape une composition de base est obtenue, composée de :
1- extraits de plantes sous forme liquide concentré en principes actifs (phytothérapie) : entre 20 et 70 % ;
2- Huiles essentielles (aromathérapie) :
entre 1 et 15 % ;
3- Eau :
entre 20 et 60 % ;
Optionnel :
4- Extraits de plantes sous forme de poudres concentrés en principes actifs (phytothérapie) ;
5- Minéraux (oligothérapie);
6- Vitamines ;
7- Huiles végétales ;
8- Plasma marin ;
9- Silicium organique (anti-agglomérant).
Dans une deuxième étape un prémix solidifiant est ajouté, composé de
- Alginates (exemple : algues brunes E401) et Calcium (sulfate de calcium) à effet gélifiant.
- Gomme(s) végétale(s) à effet émulsionnant, liant, épaississant (exemples : gomme *Karaya, Sterculia, Acacia* à *gomme* ou *Acacia senegal gum* extrait) :
Optionnel :
- Substances naturelles ou chimiques à effet retardant (exemples : chélateurs d'eau comme le *Bambusa bambou,* chélateurs de calcium comme les polyphosphates, monosaccharides)
- Plante sèche hydrophile (exemple : *Plantage ovata*) ;
- Autres substances, même chimiques, apportant aussi la gastro-résistance des actifs.

2. Complément alimentaire, obtenu par le procédé de la revendication 1, pour consommation humaine ou animale.

3. Complément alimentaire, obtenu par le procédé de la revendication 1, pour son utilisation par voie orale dans le traitement préventif des hommes ou des animaux.

4. Procédé de préparation pour compléments alimentaires selon la revendication 1, **caractérisé en ce que** le prémix solidifiant est ajouté à température ambiante.

5. Complément alimentaire, obtenu par le procédé de la revendication 1, **caractérisé en ce que** le complément alimentaire a la forme d'une gélule individuelle sous blister, de gélules en vrac dans un pot, ou a la forme d'une pâte dans un pot.

## Patentansprüche

1. Herstellungsverfahren für Nahrungsergänzungsmittel, **dadurch gekennzeichnet, dass** es mindestens die folgenden Schritte umfasst:
In einem ersten Schritt wird eine Basiszusammensetzung erhalten, bestehend aus:
1 - Pflanzenextrakten in flüssiger Form, konzentriert in aktiven Bestandteilen (Phytotherapie): zwischen 20 und 70%;
2 - essentiellen Ölen (Aromatherapie):
zwischen 1 und 15%;
3 - Wasser:
zwischen 20 und 60%;
Optional:
4 - Pflanzenextrakten in pulverisierter Form konzentriert in aktiven Bestandteilen (Phytotherapie) ;
5 - Mineralstoffen (Oligotherapie) ;
6 - Vitaminen;
7 - Pflanzenölen;
8 - Meeresplasma;
9 - organischem Silizium (Trennmittel).
In einem zweiten Schritt wird eine Verfestigungs-Vormischung zugesetzt, bestehend aus:
- Alginaten (z.B.: Braunalgen E401) und Kalzium (Kalziumsulfat) mit einer Gelierwirkung.
- pflanzlichen Verdickungsmittel(n) mit einer Emulgier-, Binde- und Verdickungswirkung (Beispiele: *Karaya gum, Sterculia,* Gummi arabicum oder *Acacia senegal gum-Extrakt,* etc.):
Optional:
- natürlichen oder chemischen Substanzen mit einer Verzögerungswirkung (beispiele: Wasserchelatoren, wie etwa *Bambusa bamboo*, Kalziumchelatoren, wie etwa Polyphosphate, Monosaccharide);
- trockener hydrophiler Pflanze (z.B.: *Plantago avata*) ;
- anderen Substanzen, sogar Chemikalien, die ebenfalls die Gastroresistenz der aktiven Substanzen bereitstellen.

2. Nahrungsergänzungsmittel, erhalten durch das Verfahren von Anspruch 1, zum Verzehr durch Mensch oder Tier.

3. Anwendung eines Nahrungsergänzungsmittels, erhalten durch das in Anspruch 1 beschriebene Verfahren, zur präventiven oder therapeutischen Behandlung von Menschen oder Tieren durch orale Verabreichung.

4. Herstellungsverfahren für Nahrungsergänzungsmittel nach Anspruch 1, **dadurch gekennzeichnet, dass** die verfestigende Vormischung bei Umgebungstemperatur zugesetzt wird.

5. Nahrungsergänzungsmittel, erhalten durch das Verfahren von Anspruch 1, dadurch gekennzeichet, dass das Nahrungsergänzungsmittel in Form einer einzelnen Kapsel in einer Blisterverpackung, loser Kapseln in einem Behälter oder in Form einer Paste in einem Gefäß vorliegt.

## Claims

1. Preparation process for dietary supplements, **characterised in that** it comprises at least the following steps:
In a first step a basic composition is obtained, consisting of:
1- Plant extracts in liquid form concentrated in active constituents (phytotherapy): between 20 and 70%;
2- Essential oils (aromatherapy):
between 1 and 15%;
3- Water:
between 20 and 60%;
Optional:
4- Plant extracts in powdered form concentrated in active constituents (phytotherapy);
5- Minerals (oligotherapy);
6- Vitamins;
7- Vegetable oils;
8- Marine plasma;
9- Organic silicon (anticaking agent).
In a second step a solidifying premix is added, consisting of:
- Alginates (e.g.: brown algae E401) and Calcium (calcium sulphate) with a gelling effect.
- Vegetable gum(s) with an emulsifying, binding and thickening effect (examples: *Karaya* gum, *Sterculia,* Gum arabic or *Acacia senegal gum* extract, etc):
Optional:
- Natural or chemical substances with a retarding effect (examples: water chelators such as *Bambusa bamboo,* calcium chelators such as polyphosphates, monosaccharides);
- Dry hydrophilic plant (e.g: *Plantago ovata*);
- Other substances, even chemicals, also providing the gastro-resistance of the active substances.

2. Dietary supplement, obtained by the process of claim 1, for human or animal consumption.

3. Use of a dietary supplement, obtained by the process described in claim 1, for the preventive or therapeutic treatment of people or animals by oral administration.

4. Preparation method for dietary supplements according to claim 1, **characterised in that** the solidifying premix is added at ambient temperature.

5. Dietary supplement, obtained by the process of claim 1, **characterised in that** the dietary supplement has the form of an individual capsule in a blister pack, loose capsules in a jar, or in the form of a paste in a pot.
